(19) Europäisches Patentamt
European Patent Office
Office européen des brevets

(11) Publication number: **0 519 877 B1**

# (12) EUROPEAN PATENT SPECIFICATION

(45) Date of publication of patent specification: **27.09.95**

(51) Int. Cl.[6]: **C07D 207/273**, C07D 207/277, C07D 401/12, A61K 31/40, A61K 31/455

(21) Application number: **92830293.4**

(22) Date of filing: **04.06.92**

(54) **3-Acylamino-2-pyrrolidinones as enhancers of the processes of learning and memory and pharmaceutical compositions containing same.**

(30) Priority: **05.06.91 IT RM910391**

(43) Date of publication of application:
**23.12.92 Bulletin 92/52**

(45) Publication of the grant of the patent:
**27.09.95 Bulletin 95/39**

(84) Designated Contracting States:
**AT BE CH DE DK ES FR GB GR LI LU NL PT SE**

(56) References cited:
**EP-A- 0 005 689**
**EP-A- 0 071 216**
**EP-A- 0 408 524**

**JOURNAL OF MEDICINAL CHEMISTRY vol. 31, no. 7, July 1988, pages 1430 - 1436; K.-L. YU ET AL.: 'DOPAMINE RECEPTOR MODULATION BY CONFORMATIONALLY CONSTRAINED ANALOGUES OF PRO-LEU-GLY-NH2'**

(73) Proprietor: **Sigma-Tau Industrie Farmaceutiche Riunite S.p.A.**
**Viale Shakespeare, 47**
**I-00144 Roma (IT)**

(72) Inventor: **Giannessi, Fabio**
**Via Abbadia San Salvatore, 16**
**I-00189 Roma (IT)**
Inventor: **Ghirardi, Orlando**
**Via Filippo de Grenet, 69**
**I-00128 Roma (IT)**
Inventor: **Misiti, Domenico**
**Via Bacchiglione, 3**
**I-00199 Roma (IT)**
Inventor: **Tinti, Maria Ornella**
**Via Ernesto Basile, 81**
**I-00182 Roma (IT)**
Inventor: **Cozzolino, Roberto**
**Piazza S. Maria alle Fornaci, 2**
**I-00165 Roma (IT)**
Inventor: **Scolastico, Carlo**
**Via Vallisneri, 13/b**
**I-20133 Milano (IT)**

EP 0 519 877 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

PEPTIDES vol. 8, 1987, pages 855 - 861; G. RAJAKUMAR ET AL.: 'DOWN-REGULATIONOF HALOPERIDOL-INDUCED STRIATAL DOPAMINE RECEPTOR SUPERSENSITIVITY BY ACTIVEANALOGUES OF L-PROLYL-L-LEUCYL-GLYCINAMIDE (PLG)'

JOURNAL OF THE CHEMICAL SOCIETY, PERKIN TRANSACTIONS II no. 1, 1989, pages 83 -87; G. VALLE ET AL.: 'CRYSTAL-STATE STRUCTURES OF BOC-PRO-LEU-GLY-NH2HEMIHYDRATE AND TWO LACTAM-RESTRICTED ANALOGUES'

JOURNAL OF THE CHEMICAL SOCIETY, PERKIN TRANSACTIONS I no. 18, 1973, pages 1934- 1940; G.J. KOOMEN ET AL.: 'UNCONVENTIONAL NUCLEOTIDE ANALOGUES. PART X. SYNTHESIS OF N-SUBSTITUTED 3-(ADENIN-9-YL)PYRROLIDIN-2-ONES'

JOURNAL OF MEDICINAL CHEMISTRY vol. 29, no. 2, February 1986, pages 251 - 260; E.D. THORSETT ET AL.: 'CONFORMATIONALLY RESTRICTED INHIBITORS OF ANGIOTENSIN CONVERTING ENZYME: SYNTHESIS AND COMPUTATIONS

(74) Representative: **Cavattoni, Fabio et al**
**Cavattoni & Raimondi**
**Viale dei Parioli, 160**
**I-00197 Roma (IT)**

## Description

The present invention relates to 3-acylamino-2-pyrrolidinones of general formula (I)

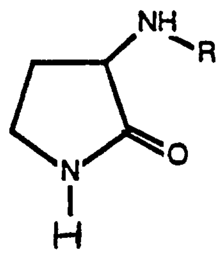

(I)

wherein R is selected from 3-trifluoromethylbenzoyl and acyl having 1-5 carbon atoms.

The compounds (I) are potent enhancers of the processes of learning and memory.

Because of the presence of one chiral carbon atom, they can exist as two enantiomers designated (R) and (S); although both enantiomers are pharmacologically active, for the sake of simplicity reference shall be made hereinbelow to the (S)-type enantiomer.

The present invention also relates to orally and parenterally administrable pharmaceutical compositions for enhancing the processes of learning and memory, comprising a novel compound of general formula (I) as active ingredient.

The known compounds that structurally and pharmacologically are most strictly related to the compound of general formula (I) are PIRACETAM [see e.g. Curr. Dev. Psychopharmacol. 3, 22, (1976] and OXIRACETAM [see e.g. Il Farmaco, Ed. Sc 39/1, 16, (1984)].

EP-A-005 689, EP-A-0 071 216 and EP-A-0 408 524 all disclose substituted pyrrolidin-2-ones as nootropic agents, which, however, bear no 3-acylamino substituents.

More specifically, EP-A-005 689 discloses alpha-hydroxy or alpha-hydroxyalkyl-2-oxo-1-pyrrolidineacetic acids or amides thereof which are optionally 3-alkyl or 3-aryl substituted.

EP-A-0 071 216 discloses 3-substituted (pyrroildin-2-one-1-yl) acetamides wherein the substituent is hydroxyl or an ester group.

EP-A-0 408 524 discloses (pyrrolidin-2-one-1-yl) acetamides, optionally 4-hydroxy substituted.

With reference to other known substituted pyrrolidin-2-ones having however pharmacological activities other than those of the compounds of the present invention. J. Med. Chem (1988) 31, 1430-1436 discloses 3(S)-and 3(R)-[[N-(benzyloxy-carbonyl)-L-prolyl]amino]-2-oxo-1-pyrrolidineacetamide as modulators of dopamine receptor activity. Peptides (1987) 8, 855-861 discloses a similar compound having an N-unprotected prolyl, whilst J. Chem. Soc., Perkin Trans. II (1989) 83-87 discloses, a similar compound wherein the N-prolyl is protected by tert-butyloxycarbonyl.

Finally, J. Med. Chem. (1986) 29, 251-260 discloses a 3-alkoxycarbonylamino (specifically 3-tertbutyloxycarbonylamino) 2-pyrrolidinone useful as intermediate in the synthesis of ACE inhibitors.

As it will be shown hereinbelow, the compounds of the present invention are more potent than the known compounds. The compounds of formula (I) are prepared by a process illustrated in the following reaction scheme.

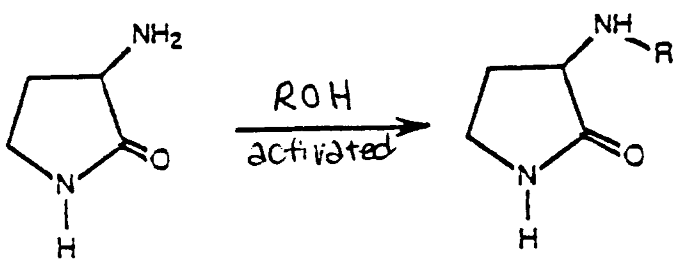


I

(S)-3-amino-2-pyrrolidinone (ST 802, prepared as disclosed in Synthesis, 614, 1978) is reacted in an anhydrous organic solvent such as acetonitrile or methylene chloride or an acetonitrile-water mixture at

room temperature, for 10-24 hours, with an equimolar amount of a carboxylic acid ROH wherein R has the aforesaid meanings activated by a halogenating agent such as thionyl or oxalyl chloride, or a condensating agent such as dicyclohexyl carbodiimide (DCC), carbonyldiimidazole (CDI), 2-ethoxy-1-ethoxycarbonyl-1,2-dihydroquinoline (EEDQ). The condensation product thus obtained is purified by crystallization or silica gel chromatography.

The following examples describe the preparation of some compounds of formula (I).

Example 1

*Preparation of (S)-3-(3-trifluoromethylbenzoylamino)-2-pyrrolidinone (ST 835)*

CDI (2.67 g; 16.48 mmoles) was added to 3-trifluoromethylbenzoic acid (2.85 g; 14.98 mmoles) in 100 mL $CH_2Cl_2$ under stirring at room temperature. After one hour, (S)-3-amino-2-pyrrolidinone (1.5 g; 14.98 mmoles) was added and the resulting solution was kept at room temperature under stirring for 20 hours. The solution was washed with 1N NaOH, 1N HCl, $H_2O$, saturated solution of NaCl and dried over anhydrous $Na_2SO_4$. Following solvent evaporation, 2 g of the product were obtained which was further purified by crystallization from EtOAc/n-hexane, giving 1.6 g of the title compound. Yield 40% (calculated on the crystallized product).

M.P. = 133-155 °C

$[\alpha]_D^{25}$ = -31.5° MeOH (C = 0.5)

TLC = silica gel Eluant = EtOAc - MeOH 9 : 1 Rf = 0.37

| Elementary analysis for $C_{12}H_{11}F_3N_2O_2$ | | | |
|---|---|---|---|
| Calculated | C 52.9 | H 4.0 | N 10.2 |
| Found | C 52.9 | H 4.07 | N 10.4 |

$^1$H NMR ($CDCl_3$): δ 8.08 (s ,1H, aromatic), 7.98 (d, 1H, aromatic) 7.8 (d, 1H, -CHN<u>H</u>CO) - 7.7 (d, 1H, aromatic), 7.5 (t, 1H, aromatic), 6.7 (s, 1H, -$CH_2$N<u>H</u>CO-), 4.7-4.6 (m, 1H, -C<u>H</u>NCO-), 3.5-3.38 (m, 2H, -C<u>H</u>$_2$NCO-), 2.82-2.7 (m, 1H, -C<u>H</u>HCHN-), 2.2-2 (m, 1H, -CH<u>H</u>CHN-) HPLC

μ Bondapack - $C_{18}$ Length = 300 mm Inner diameter = 3.9 mm Size = 10 μm

Eluant = $KH_2PO_4$ 0.05M/$CH_3CN$ (80 : 20)

Flow-rate = 1 mL/min

Permanence time = 13.38 min

Example 2

*Preparation of (S)-3-(formylamino)-2-pyrrolidinone (ST 879)*

CDI (4.08 g; 25.17 mmoles) was added to formic acid (965.5 mg; 20.97 mmoles) in 100 mL $CH_2Cl_2$ and the resulting solution was kept under stirring at room temperature for one hour. (S)-3-amino-2-pyrrolidinone (2.1 g; 20.97 mmoles) was added under stirring. After some hours, a white precipitate formed. After 24 hours, $Et_2O$ was added till complete precipitation. The precipitate was collected by filtration and chromatographed on silica gel using EtOAc - MeOH 8 : 2 as eluant. 2.03 g of product which was further purified by refluxing it with $CHCl_3$ were obtained. 1.2 g of the title compound were obtained.

Yield = 44% (calculated on 1.2 g)

M.P. = 177-179 °C

$[\alpha]_D^{25}$ = -86.25° MeOH (C = 0.56)

TLC = silica gel Eluant = EtOAc - MeOH 7 : 3 Rf = 0.4

| Elementary analysis for $C_5H_8N_2O_2$ | | | |
|---|---|---|---|
| Calculated | C 46.86 | H 6.29 | N 21.86 |
| Found | C 46.92 | H 5.76 | N 22.16 |

$^1$H NMR ($D_2O$): δ 8.15-8.05 (2s ,1H, -NC<u>H</u>O), 4.62-4.4 (m, 1H, -C<u>H</u>NHCO) - 3.48-3.3 (m, 1H, -C<u>H</u>$_2$NCO-), 2.6-2.45 (m, 1H, -C<u>H</u>HCHN-), 2.15-2 (m, 1H, -CH<u>H</u>CHN-)

HPLC
$\mu$ Bondapack - $C_{18}$ Length = 300 mm Inner diameter = 3.9 mm Size = 10 $\mu$m
Eluant = $KH_2PO_4$ 0.05M/$CH_3CN$ (95 : 5)
Flow-rate = 1 mL/min
Permanence time = 3.2 min

Assessment of the antiamnesic activity

In order to assess the antiamnesic activity the passive avoidance test in mice was used. Amnesia was brought about by administration of electroconvulsive shock (ECS) (cfr. Banfi et al., A screening method for substances potentially active on learning and memory. J. Pharmacol. Methods Vol.: 8 (4) 255-263, 1982).

Male CD1 mice (Charles River) weighting 25-26 g fed on a normal diet were used for the ECS-induced amnesia test.

The compounds were administered i.p.at doses equimolar to 9 and 0.9 mg/kg PIRACETAM. The compounds were dissolved in saline.

The apparatus for passive avoidance conditioning was a black plastic chamber (42 x 42 cm, height 40 cm) provided with a floor constructed of metal rods that could be electrified. From the front wall extended a white runway, 30 cm long and 10 cm wide provided with side walls 12 cm high, which led into the box through a guillotine door. The runway was lightened by a 60 W lamp whereas the box remained in the dark (cfr. Ader et al., Retention of passive avoidance response as a function of the intensity and duration of electric shock. Psychon. Sci., 26 (3), 125-127, 1972).

30 minutes following treatment with the compounds, the animals were placed on the runway. After one minute of adaptation, the door was raised and the time employed by the animal to enter the darkened box with all four feet was recorded.

Upon entry, the guillotine door was lowered and three seconds thereafter the rods were electrified, 0.24 mA for 2 seconds.

The mouse was then removed from the chamber and immediately administered an electroshock delivered through spring clips attached to the ears (square wave, intensity 20 mA, amplitude 0.6 msec, duration 0.5 s, frequency 50 Hz).

Immediately thereafter the animal was placed in the housing cage. Retention was assessed 24 hours later by placing the animal on the runway and again evaluating the latency in entering the chamber, using an end-point of 300 seconds (Bammer, loc.cit.).

In each experiment, two groups of animals in addition to the treated ones were used, that were defined as follows:

(1) ceiling control animals (treated with placebo and not subjected to amnesia treatment) to ensure that these animals not treated with the amnesia agent remembered the task;

(2) base-line control animals (treated with placebo and subjected to amnesia treatment) to ensure that ECS produced amnesia in the animals not treated with the compounds of the present invention.

The results of each compound under examination were expressed as percentage of amnesia reversal (AR) in order to make comparisons across the tested compounds.

AR is defined as follows:

$$AR = \frac{CIt}{CIc} \cdot 100$$

wherein CI, Comparison Index (the subscripts "t" and "c" refer to "treated" and "ceiling control", respectively) is defined by the formula

$$CI = [\Sigma Aij/Ni \bullet Nj)]100$$

wherein

- Ni is the number of animals belonging to the i-nth group (ceiling control or treated animals);
- Nj is the number of animals belonging to the j-nth group (base-line control animals); and
- Aij is a binary function that can take only the values + 1,0 or -1 depending on whether the latency time (in seconds) of an animal belonging to the i-nth group, Xi, is higher than, the same as or smaller

than the latency time (in seconds) of an animal of the j-nth group, Xj.

The sum ΣAij encompasses all the possible pairs obtained by combining each term Xi with each term Xj.

Whenever in performing the test the Comparison Index (CI) between ceiling control animals and base-line control animals, generally expected to range between 60 and 80%, turned out to be lower than 40% the data for the whole experiment were discarded.

The results are shown in Table 1.

In particular, at the dose of 9 mg/kg, PIRACETAM and ST 802 are inactive and ST 879 is poorly active (AR 9%); at the lower dose, 0.9 mg/kg, PIRACETAM is still inactive, whereas ST 802 and ST 879 were shown to be active, AR 24% and 53%, respectively. ST 835 is highly active both at 9 mg/kg (AR 58%) and 0.9 mg/kg (AR 51%).

## Table 1

### Passive avoidance following ECS-induced amnesia

The table shows the ARs of some compounds of the present invention. The number of animals (No.) and the AR of each compound tested at various dose levels equimolar to PIRACETAM are reported.

| | 9 mg/kg | | 0.9 mg/kg | |
|---|---|---|---|---|
| | No. | AR | No. | AR |
| Ceiling control group | 20 | 100 | 24 | 100 |
| Base-line control group | 40 | 0 | 48 | 0 |
| PIRACETAM | 30 | 0 | 27 | 0 |
| ST 802 | 12 | 0 | 10 | 24 |
| ST 835 | 24 | 58 | 12 | 51 |
| ST 879 | 12 | 9 | 24 | 53 |

### Behavioural profile

The behavioural profile was assessed in male CD1 mice (Charles River, Italy) weighing 22-24 g, using the Irwin test (IRWIN S., Drug screening and evaluation procedures; 136, 123-128 1962). The animals had been caged under normal conditions and kept fasting for the last 18 hours.

Following administration of the compounds, the behaviour of the animals was monitored for 6 hours.

The compounds were suspended in 10% arabic gum and orally administered at doses equimolar to 90, 23, 5.4 and 1.4 mg PIRACETAM/10mL/kg of body weight.

The animals of the control groups were administered 10% arabic gum (10 mL/kg, orally).

No compound altered, at the tested doses, the behavioural profile.

### Analgesic activity

The analgesic activity was assessed in CD1 mice (Charles River, Italy) weighing 22-24 g, utilizing the hot plate test (56 °C).

The animals, kept under normal caging conditions and kept fasting for 18 hours, were placed on the hot plate for 30, 60, 120 and 180 minutes following the oral administration of 90, 23, 5.4 and 1.4 mg/10 mL/kg equimolar to piracetam of each compound under examination.

The analgesic activity was assessed by measuring the increase (in seconds) of the time the animals continued to stay on the hot plate.

None of the tested compounds was shown to possess analgesic activity.

The compounds of the present invention are orally or parenterally administered, in any of the usual pharmaceutical forms which are prepared via the conventional procedures well-known to those persons skilled in pharmaceutical technology. These forms include solid and liquid oral unit dosage forms such as tablets, capsules, solutions and syrups as well as injectable forms such as sterile solutions for ampoules and phials.

For these pharmaceutical forms the usual solvents, diluents and excipients are used. Optionally, sweetening, flavouring and preservative agents can also be present. Examples of such agents are sodium carboxymethylcellulose, polysorbate, mannitol, sorbitol, starch, avicel, talcum and other agents which will be apparent to those skilled in the pharmaceutical technology.

The dose which is administered will be determined by the attending physician having regard to the age, weight and general conditions of the patient, utilizing sound professional judgement. Although effective results can be noticed at doses as low as 5 to 8 mg/kg of body weight daily, a dose of from 10 to 50 mg/kg of body weight is preferred. Whenever necessary, larger doses can be safely administered in view of the low toxicity of the compounds of this invention.

Therefore, pharmaceutical compositions in unit dosage form comprise from 50 to 500 mg of a compound of formula (I).

**Claims**

**Claims for the following Contracting States : AT, BE, CH, DE, DK, FR, GB, LI, LU, NL, PT, SE**

**1.** A 3-acylamino-2-pyrrolidinone of the general formula (I)

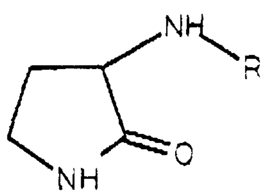

(I)

wherein R is selected from 3-trifluoromethylbenzoyl and acyl having 1-5 carbon atoms.

**2.** The pyrrolidinone of claim 1, wherein R is 3-trifluoromethylbenzoyl.

**3.** The pyrrolidinone of claim 1, wherein R is formyl.

**4.** An orally or parenterally administrable pharmaceutical composition for enhancing the processes of learning and memory, comprising as active ingredient a 3-acylamino-2-pyrrolidinone of the general formula (I)

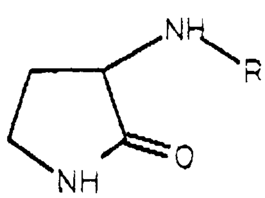

(I)

wherein R is selected from 3-trifluoromethylbenzoyl and acyl having 1-5 carbon atoms and a pharmacologically acceptable excipient therefor.

**5.** The pharmaceutical composition of claim 4 in unit dosage form, comprising from 50 to 500 mg of a compound of general formula (I).

**Claim for the following Contracting States : ES, GR**

**1.** A process for producing 3-acylamino-2-pyrrolidinones of general formula (I)

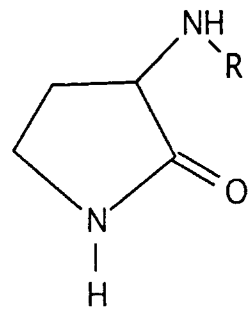

(I)

wherein R is selected from 3-trifluoromethylbenzoyl and acyl having 1-5 carbon atoms, which comprises

(a) reacting (S)-3-amino-2-pyrrolidinone in an anhydrous organic solvent such as acetonitrile or methylene chloride or an acetonitrile-water mixture at room temperature, for 10-24 hours, with an equimolar amount of a carboxylic acid ROH wherein R has the aforesaid meanings, activated by a halogenating agent such as thionyl or oxalyl chloride, or a condensating agent such as dicyclohexyl carbodiimide (DCC), carbonyldiimidazole (CDI), 2-ethoxy-1-ethoxy-carbonyl-1,2-dihydroquinoline (EEDQ); and

(b) purifying and isolating the compound of step (a) by crystallization or silica gel chromatography.

**Patentansprüche**

**Patentansprüche für folgende Vertragsstaaten : AT, BE, CH, DE, DK, FR, GB, LI, LU, NL, PT, SE**

**1.** 3-Acylamino-2-pyrrolidinon der allgemeinen Formel (I)

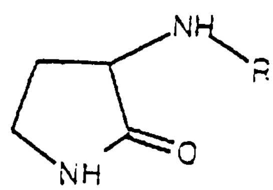

(I)

worin R aus einer 3-Trifluormethylbenzoylgruppe und einem Acylrest mit 1 bis 5 Kohlenstoffatomen ausgewählt ist.

**2.** Pyrrolidinon gemäß Anspruch 1, worin R die 3-Trifluormethylbenzoylgruppe ist.

**3.** Pyrrolidinon gemäß Anspruch 1, worin R der Formylrest ist.

**4.** Oral oder parenteral verabreichbare pharmazeutische Zusammenseztungen zur Steigerung von Lern- und Gedächtnisvorgängen, welche als Wirkstoff ein 3-Acylamino-2-pyrrolidinon der allgemeinen Formel (I)

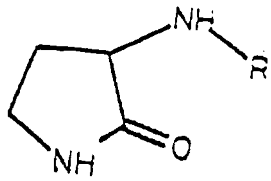

(I)

worin R aus einer 3-Trifluormethylbenzoylgruppe und einem Acylrest mit 1 bis 5 Kohlenstoffatomen ausgewählt ist, sowie einen pharmakologisch geeigneten Exzipient dafür enthalten.

**5.** Pharmazeutische Zusammensetzungen gemäß Anspruch 4 in Einheits-Dosierungsform, welche 50 bis 500 mg einer Verbindung der allgemeinen Formel (I) enthalten.

**Patentanspruch für folgende Vertragsstaaten : ES, GR**

**1.** Verfahren zur Herstellung von 3-Acylamino-2-pyrrolidine der allgemeinen Formel (I)

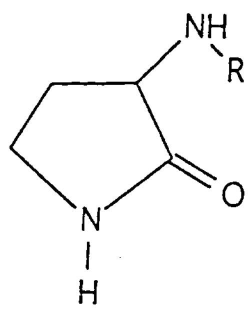

(I)

worin R aus einer 3-Trifluormethylbenzoylgruppe und einem Acylrest mit 1 bis 5 Kohlenstoffatomen ausgewählt ist, wobei man:

(a) (S)-3-Amino-2-pyrrolidinon in einem wasserfreien organischen Lösungsmittel wie Acetonitril oder Methylenchlorid oder einer Acetonitril/Wasser-Mischung bei Raumtemperatur 10 bis 24 h lang mit einer äquimolaren Menge einer Carboxylsäure ROH umsetzt, worin R die vorgenannten Bedeutungen hat, wobei die Säure mit einem Halogenierungsmittel wie Thionyl- oder Oxalylchlorid oder einem Kondensierungsmittel wie Dicyclohexylcarbodiimid (DCC), Carbonyldiimidazol (CDI), 2-Ethoxy-1-ethoxycarbonyl-1,2-dihydrochinolin (EEDQ) aktiviert ist, und

(b) die Verbindung aus Stufe (a) durch Kristallisation oder Silikagel-Chromatographie reinigt und isoliert.

**Revendications**

**Revendications pour les Etats contractants suivants : AT, BE, CH, DE, DK, FR, GB, LI, LU, NL, PT, SE**

**1.** 3-Acylamino-2-pyrrolidinone de formule générale (I)

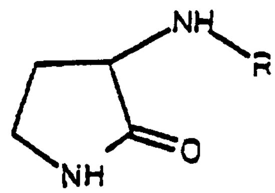

(I)

dans laquelle R est choisi parmi 3-trifluorométhylbenzoyle et acyle ayant de 1 à 5 atomes de carbone.

**2.** Pyrrolidinone selon la revendication 1, caractérisée en ce que R est 3-trifluorométhylbenzoyle.

**3.** Pyrrolidinone selon la revendication 1, caractérisée en ce que R est formyle.

**4.** Composition pharmaceutique administrable par voie orale ou parentérale pour l'amélioration de la capacité d'apprendre et de la mémoire, comprenant à titre de principe actif une 3-acylamino-2-pyrrolidinone de formule générale (I)

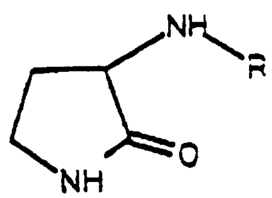

(I)

dans laquelle R est choisi parmi 3-trifluorométhylbenzoyle et acyle ayant de 1 à 5 atomes de carbone et un excipient pharmacologiquement acceptable pour celle-ci.

**5.** Composition pharmaceutique selon la revendication 4, sous forme de dose unitaire, comprenant de 50 à 500 mg d'un composé de formule générale (I).

**Revendication pour les Etats contractants suivants : ES, GR**

**1.** Procedé de production de 3-acylamino-2-pyrrolidinones de formule générale (I)

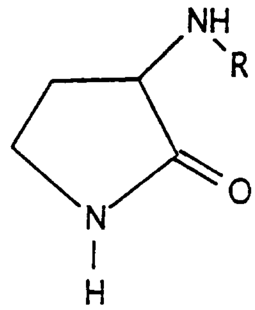

(I)

dans laquelle R est choisi parmi 3-trifluorométhylbenzoyle et acyle ayant de 1 à 5 atomes de carbone, qui comprend

(a) la réaction de (S)-3-amino-2-pyrrolidinone dans un solvant organique anhydre tel que l'acétonitrile ou le chlorure de méthylène ou un mélange acétonitrile-eau à température ambiante, pendant 10-24 heures, avec une quantité équimolaire d'un acide carboxylique ROH dans lequel R a les significations mentionnées précédemment, activé par un agent d'halogénation tel que le chlorure de thionyle ou d'oxalyle, ou un agent de condensation tel que le dicyclohexylecarbodiimide (DCC), le carbonyldiimidazole (CDI), la 2-éthoxy-1-éthoxycarbonyle-1,2-dihydroquinoléine (EEDQ); et

(b) la purification et l'isolement du composé de l'étape (a) par cristallisation ou chromatographie sur gel de silice.